(19) Europäisches Patentamt

European Patent Office

Office européen des brevets

(11) Veröffentlichungsnummer: **0 279 238 B1**

(12) **EUROPÄISCHE PATENTSCHRIFT**

(45) Veröffentlichungstag der Patentschrift: **02.01.92**

(21) Anmeldenummer: **88101162.1**

(22) Anmeldetag: **27.01.88**

(51) Int. Cl.⁵: **A61K 6/10, C08G 73/04**

(54) **Eine Aziridinverbindung enthaltendes Präparat für dentale Zwecke.**

(30) Priorität: **27.01.87 DE 3702233**

(43) Veröffentlichungstag der Anmeldung:
**24.08.88 Patentblatt 88/34**

(45) Bekanntmachung des Hinweises auf die
Patenterteilung:
**02.01.92 Patentblatt 92/01**

(84) Benannte Vertragsstaaten:
**AT CH DE FR GB IT LI**

(56) Entgegenhaltungen:
**EP-A- 0 110 429**
**DE-A- 1 544 837**
**US-A- 4 167 618**

(73) Patentinhaber: **ESPE Stiftung & Co**
**Produktions- und Vertriebs KG**
**Am Griesberg 2**
**W-8031 Seefeld(DE)**

(72) Erfinder: **Jochum, Peter, Dr.**
**Pointweg 5**
**W-8031 Seefeld 2(DE)**
Erfinder: **Zahler, Wolf-Dietrich, Dr.**
**An der Beermahd 5**
**W-8031 Seefeld-Hechendorf(DE)**
Erfinder: **Gasser, Oswald, Dr.**
**Höhenstrasse 10**
**W-8031 Seefeld(DE)**
Erfinder: **Lechner, Günter, Dr.**
**Hurtenstrasse 8**
**W-8138 Frieding(DE)**
Erfinder: **Ellrich, Klaus, Dr.**
**Auingerstrasse 16**
**W-8031 Wörthsee(DE)**

(74) Vertreter: **Abitz, Walter, Dr.-Ing. et al**
**Abitz, Morf, Gritschneder, Freiherr von Wittgenstein Postfach 86 01 09**
**W-8000 München 86(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Zur Herstellung von präzisen Abdrücken, besonders in der Zahnheilkunde, sowie von Arbeitsmodellen, insbesondere für die Zahntechnik, und von provisorischen Zahnersatzteilen werden aziridinhaltige Substanzen polymerisiert, wie sie z.B. in den US-PSen 3 453 242 und 4 093 555 beschrieben sind. Üblicherweise werden hierzu die aziridinhaltigen Verbindungen zusammen mit Füllstoffen, Farbstoffen und weiteren Hilfsstoffen verwendet.

Zur Initiierung der Polymerisationsreaktion sind die aus der US-PS 4 167 618 bekannten Sulfonisumsalze, die in $\beta$-Stellung zum zentralen Schwefelatom eine elektronenanziehende Gruppe sowie ein nicht nucleophiles Anion enthalten, geeignet.

Ferner sind aus der EP-A 0 110 429 in gelöster Form vorliegende Imidazole als Verzögerer der mit Sulfiniumsalzen initiierten Polymeristion von Aziridinverbindungen bekannt.

Es hat sich gezeigt, dass mit den bekannten Systemen die Verarbeitungszeit der initiierten Mischungen für einige Anwendungszwecke, insbesondere bei der Herstellung von besonders grossen Arbeitsmodellen oder bei hohen Aussentemperaturen, nicht ausreichen einstellbar ist. Gerade in solchen Fällen hat es sich gezeigt, dass es besonders schwierig ist, eine lange Verarbeitungszeit mit einem möglichst kurzen Abbindeübergang zu verknüpfen. Bei der Herstellung eines vollständigen Kieferabdruckes sollte die Verarbeitungszeit der initiierten Mischung beispielsweise ausreichend sein, das Material auf einen Abdrucklöffel und in eine Spritze einzubringen, die präparierten Zahnstümpfe zu umspritzen und anschliessend den gefüllten Abdrucklöffel in den Mund des Patienten zu bringen. Erst ab diesem Zeitpunkt soll die Polymerisation möglichst rasch vonstatten gehen, damit der Abdruck nach kurzer Zeit ohne Dimensionsänderung entnommen werden kann und die zeitliche Belastung für Zahnarzt und Patient erträglich bleibt. Ähnliches gilt auch bei der intraoralen Herstellung eines provisorischen Zahnersatzteils oder bei der Herstellung von Keifermodellen durch Zahntechniker.

Aufgabe der Erfindung ist die Bereitstellung von Verbindungen, die die mit Sulfoniumsalzen initiierte Polymerisationsreaktion der Aziridinverbindungen besonders wirksam verzögern, ohne hierbei den Abbindeübergang wesentlich zu verlängern.

Es wurde gefunden, dass in den Aziridinverbindungen lösbare oder dispergierbare, ionogene Verbindungen die Verarbeitungszeit von mit Sulfoniumsalzinitiatoren vermischten Aziridinverbindungen verlängern, ohne hierbei den Abbindeübergang nennenswert zu beeinflussen, sofern die ionogenen Verbindungen Anionen enthalten, welche stärker nucleophil sind als die Anionen, welche in den eingesetzten Sulfoniumsalzen vorliegen.

Gegenstand der Erfindung ist demnach ein Präparat für dentale Zwecke, enthaltend drei räumlich voneinander getrennte Bestandteile, nämlich

(a) mindestens eine Aziridinverbindung,

(b) mindestens einen Sulfoniumsalz-Initiator für (a), und

(c) mindestens eine in (a) lösbare oder dispergierbare, ionogene Verbindung, die ein Anion aufweist, das nucleophiler ist als das Anion von (b) und ausgewählt ist unter ein Fluor-, Jod-, Brom-, Chlor-, Sulfonat-, Sulfat-, Alkylsulfat-, Arylsulfat- oder Carboxylat-Anion enthaltenden Verbindungen.

Gegenstand der Erfindung ist ausserdem die Verwendung einer in Aziridinverbindungen lösbaren oder dispergierbaren, ionogenen Verbindung zur Verzögerung der durch ein Sulfoniumsalz initiierten Polymerisation von Aziridinverbindungen, wobei die ionogene Verbindung ein Anion enthält, das stärker nucleophil ist als das im verwendeten Sulfoniumsalz vorliegende Anion und ausgewählt ist unter ein Fluor-, Jod-, Brom-, Chlor-, Sulfonat-, Sulfat-, Alkylsulfat-, Arylsulfat- oder Carboxylat-Anion enthaltenden Verbindungen.

Weiter ist Gegenstand der Erfindung ein Verfahren zur Herstellung gebrauchsfertiger dentaler Abformmassen, das dadurch gekennzeichnet ist, dass

(a) mindestens eine Aziridinverbindung,

(b) mindestens ein Sulfoniumsalz-Initiator für (a) und

(c) mindestens eine in (a) lösbare oder dispergierbare, ionogene Verbindung, die ein Anion aufweist, das nucleophiler ist als das Anion von (b) und ausgewählt ist unter ein Fluor-, Jod-, Brom-, Chlor-, Sulfonat-, Sulfat-, Alkylsulfat-, Arylsulfat- oder Carboxylat-Anion enthaltenden Verbindungen.

miteinander homogen vermischt werden.

Lösbar oder dispergierbar soll heissen, dass die Verbindung (c) in (a) löslich ist oder durch übliche Massnahmen, z.B. Zusatz von Lösungsvermittlern, in (a) in Lösung oder Dispersion gebracht werden kann.

Die Einstufung der Anionen nach ihrer Nucleophilie geschieht beispielsweise wie aus E.S. Gould, "Mechanismus und Struktur in der organischen Chemie", Verlag Chemie 1962, S. 248 f. und S. 307 f. oder den Literaturstellen C.G. Swain et al, J.Am.Chem.Soc., 75, S. 141 (1953), und A.B. Ash et al., J.Org.Chem., 34, S. 4071 (1969) ersichtlich.

2

Als Aziridinverbindungen (a) können die eingangs erwähnten Verbindungen der US-PSen 3 453 242 und 4 093 555 Verwendung finden, z.B. Polyether mit Aziridino-Endgruppen und Bisphenol-A-Derivate mit Aziridino-Endgruppen.

Geeignete Sulfoniumsalzinitiatoren (b) sind in der US-PS 4 167 618 beschreiben, wobei die Sulfoniumsalze, die in $\beta$-Stellung zum S-Atom Nitril- oder Estergruppen enthalten, bevorzugt sind. Besonders bevorzugt sind Sulfoniumsalze mit Fluorborat-Anionen, die neben hoher Wirksamkeit ausgezeichnete Stabilität aufweisen. Die Menge der verwendeten Sulfoniumsalze beträgt üblicherweise 1 - 8 % bei Aziridinverbindungen, wie sie in der US-PS 4 093 555 beschrieben sind, bei anderen Aziridinverbindungen 2 - 20 %, bezogen auf das Gewicht der Aziridinverbindungen.

Die ionogenen Verbindungen (c) werden in Mengen von 0,1 bis 15 %, bezogen auf das Gewicht der Aziridinverbindungen, eingesetzt. Besonders bevorzugt sind 1 bis 10 Gew.-%.

Die ionogene Verbindung (c) liegt entweder in Substanz, vor allem wenn sie einen flüssigen Aggregatzustand aufweist, oder in gelöster oder dispergierter Form vor, besonders wenn der Schmelzpunkt über Raumtemperatur liegt. Geeignete Lösungs- oder Dispersionsmittel sind Substanzen, die in der kunststoffverarbeitenden Industrie als Weichmacher benützt werden, z.B. Phthalate und Citrate.

Die ionogene Verbindung oder deren Lösung oder Dispersion kann mit Füllstoffen oder Pigmenten und ggf. zusätzlichen Hilfsstoffen in pastenähnlicher Form vorliegen; dies ist vorteilhaft, wenn auch die Bestandteile (a) und (b) als Pasten vorliegen, da dann die Bestandteile (a) bis (c) durch die Pastenstränglänge (Volumen) dosiert und vermischt werden können.

Die Herstellung der erfindungsgemäss verwendeten ionogenen Verbindungen ist bekannt. Eine ganze Reihe von Substanzen ist käuflich erhältlich.

Durch die Verwendung des Bestandteils (c) lässt sich die mit dem Sulfoniumsalz (b) initiierte Polymerisation der Aziridinverbindung (a) erheblich verzögern. Dabei kann durch Variation der Menge von (c) die Abbindezeit von (a) stark variiert werden.

Ein weiterer Vorteil des erfindungsgemässen Präparats ergibt sich durch die Möglichkeit der Verwendung sehr reaktiver und sehr stabiler Sulfoniumsalz-Initiatoren (b). Diese besitzen vorzugsweise einen Nitrilsubstituenten in $\beta$-Stellung zum zentralen Schwefelatom und ein Fluorboratanion.

Bei Verwendung von Sulfoniumsalzen (b), die das Fluorboratanion enthalten, eignen sich als ionogene Verbindung (c) Verbindungen, die Anionen enthalten, die nucleophiler sind als das Fluorboratanion. Geeignet sind hierzu F-, J-, Br-, Cl-, Sulfonat-, Sulfat-, Alkylsulfat-, Arylsulfat- und Carboxylat-Anionen. Besonders bevorzugt sind Sulfonat-, Alkylsulfat- und Carboxylat-Anionen, insbesondere Benzolsulfonat-, Xylolsulfonat-, p-Toluolsulfonat-, Ethosulfat-, Methosulfat- und Laurinat-Anionen, sowie Fluorid, Bromid, Chlorid und Jodid.

Das Kation der Verbindung (c) erfüllt üblicherweise die Funktion, die ionogene Verbindung in den Aziridinverbindungen lösbar zu machen. Hierzu geeignete Kationen sind beispielsweise quaternäre Ammonium-und Phosphonium- oder tertiäre Sulfoniumkationen. Besonders geeignet sind quaternäre Ammoniumkationen, beispielsweise Tetrabutyl-ammonium-, Ethyl-trioctylammonium, Butyl-trioctyl-ammonium-, Ethyl-triisooctyl-ammonium- oder Ethyl-trihexyl-ammonium-Kationen.

Beispiele für geeignete ionogene Verbindungen (c) sind bei Verwendung von Fluorboratsulfoniumsalzen: Trioctyl-ethyl-ammonium-xylolsulfonat, Triisooctylmethyl-ammonium-tosylat, Trioctyl-butyl-ammonium-tosylat, Trioctyl-ethyl-ammonium-benzolsulfonat, Trioctyl-ethyl-ammonium-ethosulfat, Trioctyl-ethyl-ammonium-laurylsulfonat, Triisooctyl-ammonium-ethosulfat, Tributyl-ethyl-phosphonium-tosylat, Triisooctyl-ethyl-ammonium-trifluoracetat, Dodecylbenzolsulfonsäure-Kaliumsalz, Tetrabutyl-ammonium-laurinat, Trihexyl-ethyl-ammonium-tosylat, Trihexyl-ethyl-ammonium-benzolsulfonat, Tetrabutyl-ammonium-jodid, Tetrabutyl-ammonium-bromid, Tetrabutyl-ammonium-tosylat, Tetrabutyl-ammonium-trifluoracetat, Triisooctyl-ethyl-ammonium-p-tosylat,Tetrabutyl-phosphonium-chlorid, Phenylethyl-dibutyl-ethyl-ammonium-chlorid, Triisooctyl-methyl-ammonium-p-toluolsulfonat, $\beta$-Phenylethyl-dibutyl-methyl-ammonium-jodid, Methyl-trioctyl-ammonium-chlorid, Trioctyl-butyl-ammonium-bromid.

Bei Verwendung von Sulfoniumsalzen (b) mit p-Toluolsulfonat-, Alkylsulfat- oder Benzolsulfonat-Anionen eignen sich als Verzögerer-Substanzen (c) beispielsweise Jodid, Bromid, Chlorid u. ä. Anionen enthaltende Verbindungen.

Beispiele 1 - 6

1,0 g eines Polyethers mit Aziridino-Endgruppen, der ein durchschnittliches Molgewicht von ca. 6500 besitzt und dessen Herstellung in Beispiel 13 der US-PS 3 453 242 beschrieben ist, wird mit 0,08 g $\beta$-(S-Lauryl-S-ethyl-sulfonium)-butyronitril-fluorborat homogen vermischt. Gleichzeitig werden die in der Tabelle angegebenen Prozentsätze (Gew.-%) an ionogener Verbindung (c) zugesetzt. In Tabelle 1 findet sich in der zweiten Spalte die jeweils eingesetzte, lösbare, ionogene Verbindung, in den letzten beiden Spalten finden sich der Gelierungszeitpunkt und der Zeitpunkt, ab dem eine staubtrockene gummielastische Masse vorhanden ist (Erhärtung), gerechnet jeweils vom Zeitpunkt des Vermischens der drei Bestandteile.

Tabelle 1

| Beispiel Nr. | Ionogene Verbindung | Konzentration der ionogenen Verbindung (Gew.-%) | Zeit bis zur | |
| --- | --- | --- | --- | --- |
| | | | Gelierung (min.) | Erhärtung (min.) |
| 1 | (Vergleich) | 0 | 2,0 | 4,5 |
| 2 | Trihexyl-ethyl-ammonium-tosylat | 7 | 3,5 | 7,5 |
| 3 | Trihexyl-ethyl-ammonium-benzol-sulfonat | 5 | 3,0 | 6,5 |
| 4 | Tributyl-ethyl-phosphonium-tosylat | 4 | 3,2 | 7,2 |
| 5 | Tetrabutyl-ammonium-jodid | 3 | 3,2 | 5,2 |
| 6 | Tetrabutyl-ammonium-bromid | 2 | 4,0 | 6,3 |

Beispiele 7 - 22

Zur Herstellung einer Abdruckmasse für zahnärztliche Zwecke werden 800 g der im Beispiel 1 genannten difunktionellen Aziridinverbindung mit 350 g feinem Kieselgur verknetet, ausserdem werden 6,4 g 1-Lauryl-imidazol zugesetzt. 1 g dieser Paste werden mit 0,2 g eines 1:1 Gemisches aus Acetyl-tributyl-citrat und dem im Beispiel 1 genannten Sulfoniumsalz vermischt. Ausserdem werden die in der Tabelle 2 angegebenen Gew.-% (bezogen auf die eingesetzte Menge an Aziridinverbindungen) an ionogener Verbindung (c) zugesetzt.

4

TABELLE 2

| Beispiel Nr. | Ionogene Verbindung | Konzentration der ionogenen Verbindung (Gew.-%) | Zeit bis zur Gelierung (min.) | Zeit bis zur Erhärtung (min.) |
|---|---|---|---|---|
| 7 | (Vergleich) | 0 | 3,0 | 5,5 |
| 8 | Trioctyl-ethyl-ammonium-ethosulfat | 5 | 4,0 | 8,3 |
| 9 | Tetrabutyl-ammonium-tosylat | 5 | 4,0 | 7,5 |
| 10 | Tetrabutyl-ammonium-trifluoracetat | 1 | 3,5 | 6,0 |
| 11 | Triisooctyl-methyl-ammonium-tosylat | 5 | 4,0 | 8,0 |
| 12 | Trioctyl-ethyl-ammonium-benzolsulfonat | 5 | 3,6 | 7,2 |
| 13 | Tributyl-ethyl-phosphonium-tosylat | 3 | 3,7 | 7,2 |
| 14 | Triisooctyl-ethyl-ammonium-p-tosylat | 3 | 3,8 | 7,5 |
| 15 | Tetrabutyl-phosphonium-chlorid | 1 | 4,0 | 7,2 |
| 16 | Phenylethyl-dibutyl-ethyl-ammonium-chlorid | 1 | 4,0 | 6,7 |
| 17 | Tetrabutyl-ammonium-jodid | 3 | 4,25 | 7,6 |
| 18 | Tetrabutyl-ammonium-bromid | 1 | 3,75 | 6,5 |
| 19 | Triisooctyl-methyl-ammonium-p-toluolsulfonat | 4 | 4,0 | 7,7 |
| 20 | β-Phenylethyl-dibutyl-methyl-ammonium-jodid | 2 | 4,0 | 7,6 |
| 21 | Methyl-trioctyl-ammonium-chlorid | 1,5 | 4,0 | 6,7 |
| 22 | Trioctyl-butyl-ammonium-bromid | 2 | 4,0 | 7,0 |

**Patentansprüche**

1. Präparat für dentale Zwecke, enthaltend drei räumlich voneinander getrennte Bestandteile, nämlich
   (a) mindestens eine Aziridinverbindung,
   (b) mindestens einen Sulfoniumsalz-Initiator für (a), und
   (c) mindestens eine in (a) lösbare oder dispergierbare, ionogene Verbindung, die ein Anion aufweist, das nucleophiler ist als das Anion von (b) und ausgewählt ist unter ein Fluor-, Jod-, Brom-, Chlor-, Sulfonat-, Sulfat-, Alkylsulfat-, Arylsulfat- oder Carboxylat-Anion enthaltenden Verbindungen.

2. Präparat nach Anspruch 1, dadurch gekennzeichnet, daß es als (b) eine ein Fluorboratanion enthaltende Verbindung enthält.

3. Präparat nach Anspruch 2, dadurch gekennzeichnet, daß es als (c) eine ein Benzolsulfonat-, Xylolsulfonat-, p-Toluolsulfonat-, Ethosulfat-, Methosulfat- oder Laurinat-Anion enthaltende Verbindung enthält.

4. Verwendung einer in Aziridinverbindungen lösbaren oder dispergierbaren, ionogenen Verbindung zur Verzögerung der durch ein Sulfoniumsalz initiierten Polymerisation von Aziridinverbindungen, wobei die ionogene Verbindung ein Anion enthält, das stärker nucleophil ist als das im verwendeten Sulfoniumsalz vorliegende Anion und ausgewählt ist unter ein Fluor-, Jod-, Brom-, Chlor-, Sulfonat-, Sulfat-, Alkylsulfat-, Arylsulfat- oder Carboxylat-Anion enthaltenden Verbindungen.

5. Verfahren zur Herstellung gebrauchsfertiger dentaler Abformmassen, dadurch gekennzeichnet, daß
   (a) mindestens eine Aziridinverbindung,
   (b) mindestens ein Sulfoniumsalz-Initiator für (a) und
   (c) mindestens eine in (a) lösbare oder dispergierbare, ionogene Verbindung, die ein Anion aufweist, das nucleophiler ist als das Anion von (b) und ausgewählt ist unter ein Fluor-, Jod-, Brom-, Chlor-, Sulfonat-, Sulfat-, Alkylsulfat-, Arylsulfat- oder Carboxylat-Anion enthaltenden Verbindungen
   miteinander homogen vermischt werden.

**Claims**

1. A preparation for dental purposes containing three constituents spatially separated from each other, namely
   (a) at least one aziridine compound,
   (b) at least one sulphonium salt initiator for (a), and
   (c) at least one ionogenic compound soluble or dispersible in (a) and having an anion that is more nucleophilic than the anion of (b) and selected from compounds containing a fluoro-, iodo-, bromo-, chloro-, sulphonate, sulphate, alkylsulphate, arylsulphate or carboxylate anion.

2. Preparation according to claim 1, characterized in that it contains, as (b), a compound containing a fluoroborate anion.

3. Preparation according to claim 2, characterized in that it contains, as (c), a compound containing a benzenesulphonate, xylenesulphonate, p-toluenesulphonate, ethosulphate, methosulphate or laurinate anion.

4. Use of an ionogenic compound, soluble or dispersible in aziridine compounds, for retarding the polymerization of aziridine compounds initiated by a sulphonium salt, the ionogenic compound containing an anion that is more nucleophilic than the anion present in the sulphonium salt used and being selected from compounds containing a fluoro-, iodo-, bromo-, chloro-, sulphonate, sulphate, alkylsulphate, arylsulphate or carboxylate anion.

5. Process for preparing dental compositions ready for use, characterized in that
   (a) at least one aziridine compound,
   (b) at least one sulphonium salt initiator for (a), and

(c) at least one ionogenic compound soluble or dispersible in (a) and having an anion that is more nucleophilic than the anion of (b) and selected from compounds containing a fluoro-, iodo-, bromo-, chloro-, sulphonate, sulphate, alkylsulphate, arylsulphate or carboxylate anion

are homogeneously mixed together.

**Revendications**

1. Préparation à buts dentaires comprenant trois composants spatialement séparés l'un de l'autre, à savoir

   (a) au moins un composé d'aziridine,

   (b) au moins un inducteur sel de sulfonium pour (a), et

   (c) au moins un composé ionogène soluble ou dispersable dans (a), qui présente un anion qui est plus nucléophile que l'anion de (b) et qui est choisi parmi les composés contenant un anion fluorure, iodure, bromure, chlorure, sulfonate, sulfate, alkylsulfate, arylsulfate ou carboxylate.

2. Préparation selon la revendication 1, caractérisée en ce qu'elle contient comme (b) un composé contenant un anion fluoroborate.

3. Préparation selon la revendication 2, caractérisée en ce qu'elle contient comme (c) un composé contenant un anion benzènesulfonate, xylènesulfonate, p-toluènesulfonate, éthosulfate, méthosulfate ou laurate.

4. Utilisation d'un composé ionogène soluble ou dispersable dans les composés d'aziridine dans le retardement de la polymérisation du composé d'aziridine induite par un sel de sulfonium, où le composé ionogène contient un anion qui est plus nucléophile que l'anion présent dans le sel de sulfonium utilisé et qui est choisi parmi les composés contenant un anion fluorure, iodure, bromure, chlorure, sulfonate, sulfate, alkylsulfate, arylsulfate ou carboxylate.

5. Procédé de préparation de masses à modeler dentaires, caractérisé en ce qu'on mélange de façon homogène

   (a) au moins un composé d'aziridine,

   (b) au moins un inducteur sel de sulfonium pour (a) et

   (c) au moins un composé ionogène soluble ou dispersable dans (a), qui présente un anion qui est plus nucléophile que l'anion de (b) et est choisi parmi les composés contenant un anion fluorure, iodure, bromure, chlorure, sulfonate, sulfate, alkylsulfate, arylsulfate ou carboxylate.